# EUROPEAN PATENT SPECIFICATION

(11) **EP 3 895 757 B1**
(45) Date of publication and mention of the grant of the patent: **07.06.2023**
(21) Application number: 20182281.4
(22) Date of filing: 25.06.2020
(51) Int. Cl.: A61N 1/05, A61N 1/36, A61G 15/16

(54) **IMPROVED ELECTROTHERAPY DEVICE**
VERBESSERTE ELEKTROTHERAPIEVORRICHTUNG
DISPOSITIF D'ÉLECTROTHÉRAPIE AMÉLIORÉ

(30) Priority: 15.04.2020 ES 202030637 U
(43) Date of publication of application: 20.10.2021
(73) Proprietor: NV Gymna Uniphy, 3740 Bilzen (BE)
(72) Inventor: ABAT GONZALEZ, Ferran, 08030 Barcelona (ES)
(74) Representative: Gevers Patents

(56) References cited:
- WO-A1-01/39829
- WO-A1-2014/207283
- WO-A1-2015/144952
- ES-A1- 2 546 651
- US-A- 3 774 773
- US-A- 5 997 297
- US-A1- 2012 112 018
- US-A1- 2013 153 723
- US-A1- 2016 367 326
- US-A1- 2019 307 629
- US-B1- 6 705 474

## Description

The present invention, as set out in the appended claims, generally relates to an improved electrotherapy device for treating musculoskeletal injuries based on percutaneous electrolysis, the device being of the type that includes a single needle arranged in a needle housing, the needle acting as a cathode, a surface application electrode, which can be hand-held or attached to the patient's skin, acting as an anode, a unit for generating and controlling electric current, and the patient's tissue being the electrolyte.

This type of device is well known in the art and conventionally applies a galvanic current percutaneously to a soft tissue to be treated in a patient, causing a local inflammatory response necessary for tissue regeneration and subsequent recovery, for example, in case of injuries such as tendinopathies or injuries to muscles, tendons, ligaments or fasciae.

Thus, for example, documents ES2674804, ES2583159 or ES2546651 describe devices that allow these types of therapies to be performed. Further electrotherapy devices are shown in WO 2014/207283 A1 and WO 01/39829 A1.

The device of the present invention is defined in the independent claim, and further embodiments thereof in the dependent claims. The device and the further embodiment have a number of specific advantages in comparison with already known devices, including:
- The unit for generating and controlling electric current has a support for the needle housing that is reversible, facilitating use by left-handed users.
- The needle housing has in its head a mechanism for locking the needle to facilitate its insertion and removal, so that, in use, the necessary pressure on the needle is maintained without affecting its necessary elasticity.
- The head of the needle housing and the housing itself are protected against the ingress of liquids (for example blood or disinfectants) and can be disinfected safely.
- The head of the needle housing includes a needle guide cone for introducing the needle into the head.
- The needle housing includes means that collaborate with corresponding means in the unit for generating and controlling electric current in order to keep the needle housing in place in its support, thus minimising piercing accidents.
- The head of the needle housing has a mechanism for locking the needle using a spring and hammer.
- The needle housing has means for controlling and/or adjusting the treatment parameters, such as changes in the intensity of current applied) in the housing itself, avoiding the need to access the unit for generating and controlling electric current to modify said parameters, as well as means for displaying the status of said generation and control unit (unit status on/off, standby).

For this purpose, the improved electrotherapy device for treating musculoskeletal injuries based on percutaneous electrolysis, is of the type that includes a single needle arranged in a needle housing, the needle acting as a cathode, a surface application electrode, which can be hand-held or attached to the patient's skin, acting as an anode, a unit for generating and controlling electric current, and the patient's tissue acting as the electrolyte when the device is being used, has the following advantageous elements: a reversible support for the needle housing in the unit for generating and controlling electric current and fixation means between said needle housing and said unit for generating and controlling electric current, a head of the needle housing which includes a mechanism for locking the needle, a cone for guiding the needle and a protection against the ingress of liquids and means for adjusting and displaying the treatment parameters in the needle housing itself.

Below, the device of the invention is described on the basis of an embodiment of the same and with reference to the attached figures, in which:
- Figure 1:: is a schematic top view of the device of the invention showing the unit for generating and controlling electric current and the needle housing;
- Figure 2:: is a schematic rear view of the device of figure 1;
- Figure 3:: is a schematic view of the support for the needle housing;
- Figure 4:: is a schematic view of the needle housing
- Figure 5:: is a schematic representation of the head;
- Figure 6:: is a sectional view of the head;
- Figure 7:: is a diagram of the movement of the system for locking the needle.

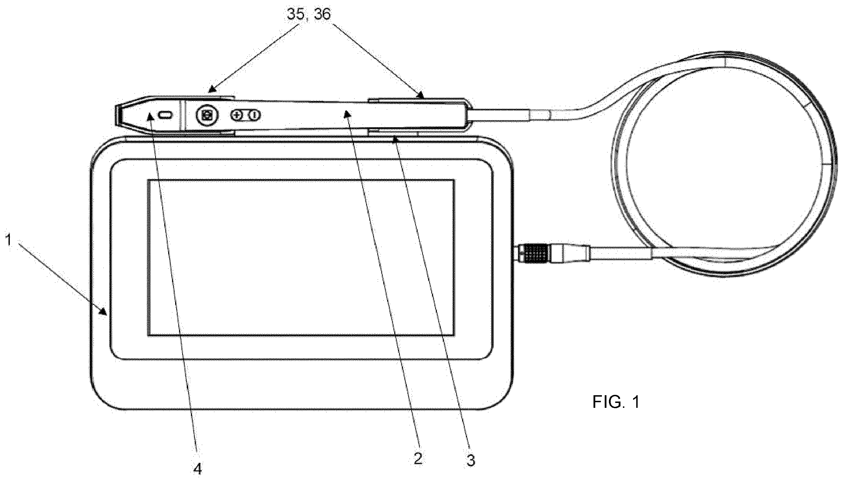

As mentioned above and shown in Figure 1, the invention is comprised of a unit for generating and controlling electric current (1) (hereinafter control unit (1)), a needle housing (2) and a surface application electrode (not shown).

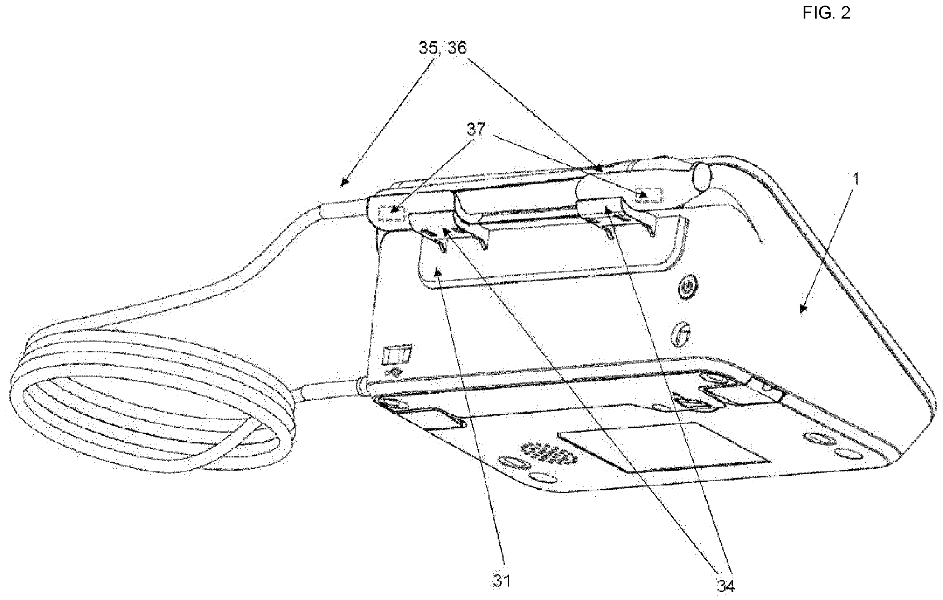

With reference to figures 1 and 2, the electric current control unit (1) has, on its rear side, a support (3) for the needle housing (2).

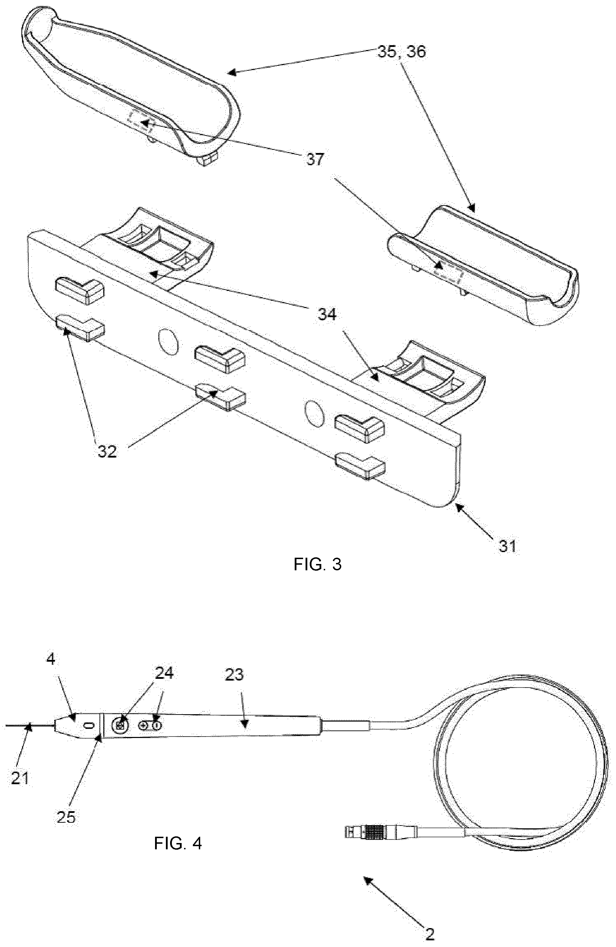

As mentioned above and shown in Figure 3, the support (3) is reversible, for which purpose it is comprised at its base of a connecting element (31) to the control unit (1). This connecting element (31) is coupled by a suitable means to said control unit (1), for example by form-fitting cooperation with a complementary means arranged in the latter. In the embodiment shown, and as a non-limitative example, the connecting element (31) has tabs (32) on one of its sides for form-fitting engagement with recesses (33) present in the control unit (1) (not shown).

Said connecting element (31) also includes at least two projecting fins (34) which are coupled in a separable manner to two support elements (35, 36), these support elements (35, 36) serving to support the needle housing (2). For this purpose, the shape of the support elements (35, 36) is matched in each case to the front and rear side of the needle housing (2), these support elements (35, 36) being interchangeable with each other.

As shown in Figure 3, the support elements (35, 36) each include magnets (37) which cooperate with corresponding magnets (not shown) arranged in the interior of the needle housing (2), which will be referred to later.

Figure 4 shows a schematic view of the needle housing (2), including a needle (21) and consisting of a head (4) and a body (23).

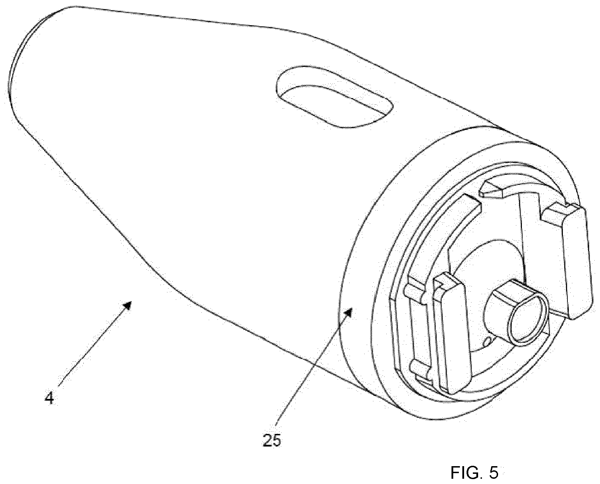

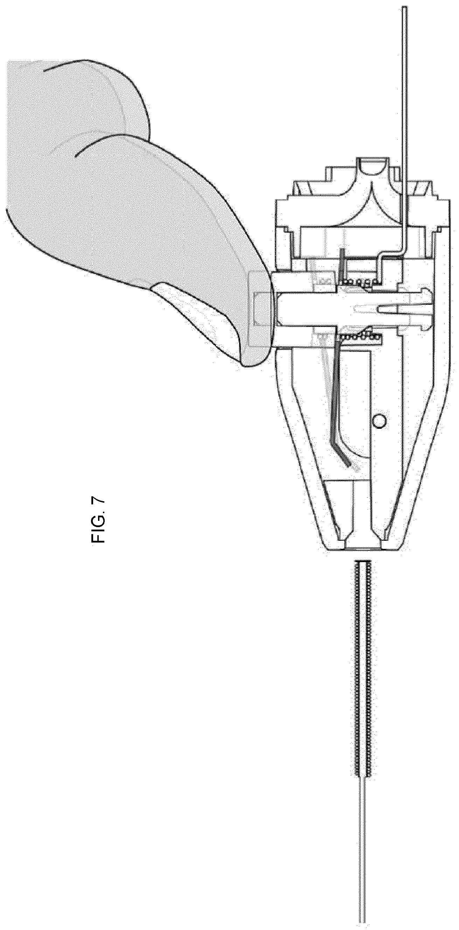

As can be seen in Figures 4 and 5, the body (23) of the needle housing (2) is cylindrical in shape and ends in the head (4), where the needle (21) is housed. The head (4), which is shown in detail in Figures 5, 6 and 7, includes in its interior a system for locking the needle (21), said system being comprised of a spring (41) and a hammer (42) which are associated with an actuating button (43) protruding from the head (4). This locking system facilitates the insertion and removal of the needle (21) so that, in use, the necessary pressure on the needle is maintained without affecting its necessary elasticity.

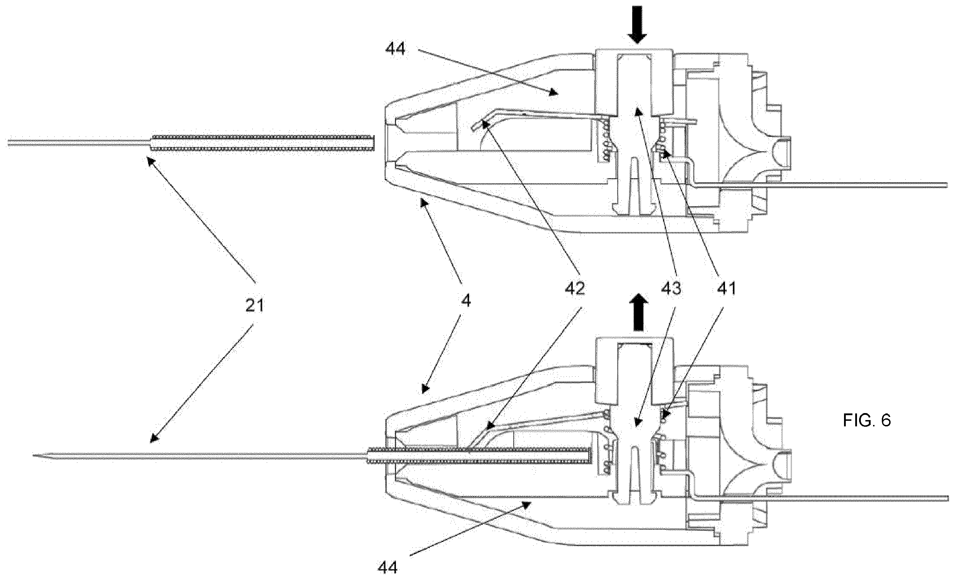

A hollow guide cone (44) is also arranged in the interior of the head (4) to make it easier for the user to introduce the needle (21) into the head (4) (Figure 6).

As shown in Figure 4, the body (23) includes buttons for controlling and/or adjusting (24) the treatment parameters, which are protected against the ingress of moisture by means of silicone or rubber elements (not shown).

With reference to Figure 3 again, inside the body (23) magnets (not shown) are arranged which correspond to the magnets (37) arranged in the support elements (35, 36) such that the needle housing (2) is held securely in its support (3).

The needle housing (2) includes means for displaying (25) the status of the generation and control unit (unit status on/off, standby). In a particular embodiment, these display means (25) are arranged downstream of the head (4) and may comprise, for example, RGB LEDs.

The display means (25) make it possible to check that the device is operational without having to look away from the treatment area, providing a higher level of safety for both the user and the patient. For example, in the case of RGB LEDs, the display modes can consist of a slow blink, a rapid blink, a steady green light, a steady orange light, a red light or similar, which are in each case associated with an indication of the progress of the treatment process.

Conventionally, the control unit (1) includes a processor that makes it possible to apply defined treatment intensities, different therapy conditions, therapy statuses such as start of treatment, temporary interruption of treatment, continuation of treatment after an interruption, end of treatment, which can be managed directly with the control and/or adjustment buttons (24) present on the body (23) of the needle housing (2).

Furthermore, the control unit (1) includes a USB or Bluetooth connection, for example for updating a therapy, adding new treatments to a database stored in the memory, extracting data for analysis (patient, treatments saved) or for restoring previously extracted data, and as a port for SD cards, a real-time clock with CR2032 battery, removable rechargeable batteries, a medical-grade battery and power supply enabling the device to be used while charging, and a capacitive screen for selecting treatment variables and displaying said variables.

## Claims

1. Electrotherapy device, the device being of the type that includes a single needle (21) arranged in a needle housing (2) and the needle housing (2) being comprised of a head (4) and a body (23), the needle acting as a cathode, a surface application electrode, which can be hand-held or attached to the patient's skin, acting as an anode, a unit for generating and controlling electric current (1), **characterised in that** the unit for generating and controlling electric current (1) having, on its rear side, a support (3) for the needle housing (2), wherein the support (3) is comprised at its base of a connecting element (31) to the unit for generating and controlling electric current (1) and which is coupled to said unit for generating and controlling electric current (1) and includes at least two projecting fins (34) which are coupled in a separable manner to two support elements (35, 36), these support elements (35, 36) serving to support the needle housing (2) and these support elements (35, 36) being interchangeable with each other such that the support (3) is reversible, wherein the shape of the support elements (35, 36) is matched in each case to the front and rear side of the needle housing (2).

2. Electrotherapy device according to claim 1, **characterised in that** the support elements (35, 36) include magnets (37) which cooperate with corresponding magnets arranged inside the needle housing (2).

3. Electrotherapy device according to claim 1 or 2, **characterised in that** the head (4) includes in its interior a system for locking the needle (21), said system being comprised of a spring (41) and a hammer (42) which are associated with an actuating button (43) protruding from the head (4).

4. Electrotherapy device according to any one of the claims 1-3, **characterised in that** the interior of the head (4) has a hollow guide cone (44) to make it easier for the user to introduce the needle (21) into the head (4).

5. Electrotherapy device according to any one of the claims 1-4, **characterised in that** the body (23) includes buttons for controlling and/or adjusting (24) the treatment parameters, which are protected against the ingress of moisture by means of silicone or rubber elements.

6. Electrotherapy device according to any one of the claims 1-5, **characterised in that** inside the body (23) magnets are arranged which correspond to the magnets (37) arranged in the support elements (35, 36) such that the needle housing (2) is held securely in its support (3).

7. Electrotherapy device according to any one of the claims 1-6, **characterised in that** the needle housing (2) includes means for displaying (25) the status of the unit for generating and controlling electric current (1).

8. Electrotherapy device according to claim 7, **characterised in that** the display media (25) are arranged downstream of the head (4) and are RGB LEDs.

9. Electrotherapy device according to any one of the claims 1-8, **characterised in that** the unit for generating and controlling electric current (1) includes USB or Bluetooth connection media, a port for SD cards, a real-time clock with CR2032 battery, removable rechargeable batteries, a medical-grade battery and power supply and a capacitive screen for selecting treatment variables and displaying said variables.

## Patentansprüche

1. Elektrotherapievorrichtung, wobei die Vorrichtung von dem Typ ist, der eine einzelne Nadel (21) einschließt, angeordnet in einem Nadelgehäuse (2), und wobei das Nadelgehäuse (2) einen Kopf (4) und einen Körper (23) umfasst, wobei die Nadel als Kathode fungiert, eine Elektrode zur Oberflächenanwendung, die mit der Hand gehalten oder an der Haut des Patienten angebracht werden kann, die als Anode fungiert, eine Einheit zum Erzeugen und Regeln von elektrischem Strom (1), **dadurch gekennzeichnet, dass** die Einheit zum Erzeugen und Regeln von elektrischem Strom (1) auf ihrer Rückseite eine Halterung (3) für das Nadelgehäuse (2) hat, wobei die Halterung (3) an ihrer Basis einen Verbindungselement (31) zur Einheit zum Erzeugen und Regeln von elektrischem Strom (1) umfasst und das mit der erwähnten Einheit zum Erzeugen und Regeln von elektrischem Strom (1) verbunden ist und zumindest zwei vorragende Lamellen (34) einschließt, die auf abtrennbare Weise mit zwei Halteelementen (35, 36) verbunden sind, wobei diese Halteelemente (35, 36) dazu dienen, das Nadelgehäuse (2) zu halten, und diese Halteelemente (35, 36) miteinander austauschbar sind, sodass die Halterung (3) umdrehbar ist, wobei die Form der Halteelemente (35, 36) in jedem Fall auf die Vorder- und Rückseite des Nadelgehäuses (2) abgestimmt ist.

2. Elektrotherapievorrichtung nach Anspruch 1, **dadurch gekennzeichnet, dass** die Halteelemente (35, 36) Magnete (37) umfassen, welche mit zugehörigen Magneten, die innerhalb des Nadelgehäuses (2) angebracht sind, zusammenwirken.

3. Elektrotherapievorrichtung nach Anspruch 1 oder 2, **dadurch gekennzeichnet, dass** der Kopf (4) in seinem Inneren ein System zum Festklemmen der Nadel (21) einschließt, wobei das erwähnte System eine Feder (41) und einen Hammer (42) umfasst, die mit einem aus dem Kopf (4) herausragenden Betätigungsknopf (43) assoziiert sind.

4. Elektrotherapievorrichtung nach irgendeinem der Ansprüche 1 bis 3, **dadurch gekennzeichnet, dass** das Innere des Kopfes (4) einen hohlen Führungskonus (44) hat, um es für den Benutzer einfacher zu machen, die Nadel (21) in den Kopf (4) einzuführen.

5. Elektrotherapievorrichtung nach irgendeinem der Ansprüche 1 bis 4, **dadurch gekennzeichnet, dass** der Körper (23) Tasten zum Regeln und/oder Anpassen (24) der Behandlungsparameter einschließt, die mittels Silikon- oder Gummielementen gegen das Eindringen von Feuchtigkeit geschützt sind.

6. Elektrotherapievorrichtung nach irgendeinem der Ansprüche 1 bis 5, **dadurch gekennzeichnet, dass** innerhalb des Körpers (23) Magnete angeordnet sind, die den in den Halteelementen (35, 36) angeordneten Magneten (37) zugehörig sind, sodass das Nadelgehäuse (2) sicher in seiner Halterung (3) gehalten wird.

7. Elektrotherapievorrichtung nach irgendeinem der Ansprüche 1 bis 6, **dadurch gekennzeichnet, dass** das Nadelgehäuse (2) Mittel zum Anzeigen (25) des Status der Einheit zum Erzeugen und Regeln von elektrischem Strom (1) einschließt.

8. Elektrotherapievorrichtung nach Anspruch 7, **dadurch gekennzeichnet, dass** die Anzeigemedien (25) dem Kopf (4) nachgelagert angeordnet sind und RGB-LEDs sind.

9. Elektrotherapievorrichtung nach irgendeinem der Ansprüche 1 bis 8, **dadurch gekennzeichnet, dass** die Einheit zum Erzeugen und Regeln von elektrischem Strom (1) USB- oder Bluetooth-Verbindungsmedien, einen Anschluss für SD-Karten, eine Echtzeituhr mit CR2032-Batterie, herausnehmbare aufladbare Batterien, eine Batterie und Stromversorgung in medizinischer Güte und ein kapazitives Display zum Auswählen von Behandlungsvariablen und zum Anzeigen der erwähnten Variablen einschließt.

## Revendications

1. Dispositif d'électrothérapie, le dispositif étant du type qui inclut une seule aiguille (21), agencée dans un logement d'aiguille (2) et le logement d'aiguille (2) étant compris d'une tête (4) et d'un corps (23), l'aiguille servant de cathode, une électrode d'application en surface, qui peut être tenue dans la main ou attachée à la peau du patient, servant d'anode, une unité de génération et de commande de courant électrique (1), **caractérisé en ce que** l'unité de génération et de commande de courant électrique (1) présente, sur son côté arrière, un support (3) pour le logement d'aiguille (2), dans lequel le support (3) est compris, à sa base, d'un élément de connexion (31) à l'unité de génération et de commande de courant électrique (1) et qui est couplé à ladite unité de génération et de commande de courant électrique (1) et inclut au moins deux ailettes saillantes (34) qui sont couplées de manière séparable à deux éléments de support (35, 36), ces éléments de support (35, 36) servant à supporter le logement d'aiguille (2) et ces éléments de support (35, 36) étant interchangeables l'un avec l'autre de telle sorte que le support (3) soit réversible, dans lequel la forme des éléments de support (35, 36) est assortie dans chaque cas au côté avant et arrière du logement d'aiguille (2).

2. Dispositif d'électrothérapie selon la revendication 1, **caractérisé en ce que** les éléments de support (35, 36) incluent des aimants (37) qui coopèrent avec des aimants correspondants agencés à l'intérieur du logement d'aiguille (2).

3. Dispositif d'électrothérapie selon la revendication 1 ou 2, **caractérisé en ce que** la tête (4) inclut, dans son espace intérieur, un système pour verrouiller l'aiguille (21), ledit système étant compris d'un ressort (41) et d'un marteau (42) qui sont associés à un bouton d'actionnement (43) faisant saillie à partir de la tête (4).

4. Dispositif d'électrothérapie selon l'une quelconque des revendications 1 à 3, **caractérisé en ce que** l'espace intérieur de la tête (4) présente un cône de guidage creux (44) pour rendre plus facile l'introduction, par l'utilisateur, de l'aiguille (21) dans la tête (4).

5. Dispositif d'électrothérapie selon l'une quelconque des revendications 1 à 4, **caractérisé en ce que** le corps (23) inclut des boutons de commande et/ou d'ajustement (24) des paramètres de traitement qui sont protégés contre l'entrée d'humidité au moyen d'éléments en silicone ou caoutchouc.

6. Dispositif d'électrothérapie selon l'une quelconque des revendications 1 à 5, **caractérisé en ce que**, à l'intérieur du corps (23), des aimants sont agencés qui correspondent aux aimants (37) agencés dans les éléments de support (35, 36) de telle sorte que le logement d'aiguille (2) soit retenu solidement dans son support (3).

7. Dispositif d'électrothérapie selon l'une quelconque des revendications 1 à 6, **caractérisé en ce que** le logement d'aiguille (2) inclut des moyens d'affichage (25) de l'état de l'unité de génération et de commande de courant électrique (1).

8. Dispositif d'électrothérapie selon la revendication 7, **caractérisé en ce que** les moyens d'affichage (25) sont agencés en aval de la tête (4) et sont des LEDs R-V-B.

9. Dispositif d'électrothérapie selon l'une quelconque des revendications 1 à 8, **caractérisé en ce que** l'unité de génération et de commande de courant électrique (1) inclut des moyens de connexion USB ou Bluetooth, un port pour cartes SD, une horloge temps-réel avec une pile CR2032, des piles rechargeables amovibles, une batterie et alimentation électrique de qualité médicale et un écran capacitif pour sélectionner des variables de traitement et afficher lesdites variables.
